# FASCICULE DE BREVET EUROPEEN

(11) **EP 2 371 307 B1**
(45) Date de publication et mention de la délivrance du brevet: **26.06.2013**
(21) Numéro de dépôt: 11158753.1
(22) Date de dépôt: 18.03.2011
(51) Int. Cl.: A61B 17/225

(54) **Appareil de traitement avec un générateur d'ondes de pression mobile**
Verarbeitungsgerät mit einem mobilen Druckwellengenerator
Treatment device with a mobile pressure wave generator

(30) Priorité: 30.03.2010 FR 1052317
(43) Date de publication de la demande: 05.10.2011
(73) Titulaire: EDAP TMS France, 69120 Vaulx-en-Velin (FR)
(72) Inventeur: Nallet, Olivier, 69003 Lyon (FR); Peyrard, André, 42320 Saint Christo en Jarez (FR)
(74) Mandataire: Thibault, Jean-Marc

(56) Documents cités:
- DE-A1- 10 303 462
- US-A- 5 329 926
- US-A- 5 468 214

## Description

La présente invention concerne un appareil de destruction de concrétions intracorporelles solides pour des traitements de lithotritie.

De manière connue, un appareil de lithotritie comporte un générateur d'ondes de pression telles que des ondes de choc acoustiques permettant de détruire des concrétions ou des lithiases rénales, biliaires ou salivaires. Un appareil de lithotritie est associé à un dispositif d'imagerie par rayons « X » ou par ultrasons permettant de détecter et de visualiser la présence de lithiases dans le corps d'un patient ou d'un animal. Les lithiases et le foyer du générateur sont mis en coïncidence à l'aide de systèmes de déplacement mécanique permettant d'amener soit les lithiases sur le point de focalisation du générateur, soit le point de focalisation du générateur sur les lithiases. De façon complémentaire, un appareil de lithotritie est associé à une table de traitement qui, de façon avantageuse, comporte une découpe pour le logement du générateur de manière que ce générateur puisse se trouver, pour être efficace, au contact du patient tout en permettant au patient d'être allongé sur la table sans déborder de la table.

Un tel équipement de lithotritie à savoir un appareil de traitement associé à son dispositif d'imagerie et à une table de traitement est largement répandu dans les centres hospitaliers. Dans diverses occasions, il apparaît le besoin d'utiliser une partie de cet équipement pour assurer des traitements autres que des traitements de lithotritie tels que par exemple des actes d'endourologie. Ces actes consistent par exemple à introduire des sondes dans les voies urinaires sous guidage des rayons « X » pour atteindre la vessie ou les reins afin de réaliser des gestes médicaux.

Cependant, les actes endourologiques ne nécessitent pas la présence du générateur d'ondes de choc réservé à la lithotritie. Compte tenu de son positionnement à l'intérieur de la découpe aménagée dans la table, le générateur provoque une gêne pour ces actes d'endourologie. Sur certains appareils de lithotritie sophistiqués, le générateur d'ondes de choc est déplacé pour être positionné dans une position de retrait en vue de faciliter l'accès au patient. Toutefois, compte tenu de la complexité et de la compacité des appareils de lithotritie, les courses de déplacement du générateur sont généralement limitées à quelques centimètres de sorte que si le générateur n'est plus au contact du patient, il reste toutefois dans le champ d'intervention de l'équipe médicale gênant les actes d'endourologie et exposant par ailleurs le générateur à des risques d'endommagement.

Dans le même sens, lors des procédures d'endourologie, certaines équipes médicales préfèrent dissocier l'appareil de lithotritie de la table support du patient et l'éloigner de cette table afin de le placer à l'extérieur de la salle de traitement. Un tel appareil de lithotritie est alors exposé à des risques de mauvaise manipulation et à des heurts par d'autres équipements médicaux susceptibles d'affecter son intégrité.

Dans l'état de la technique, il est connu par le brevet US 5 329 926, un appareil de traitement par ondes de pression comportant un générateur d'ondes de pression monté par l'intermédiaire d'une structure de guidage, sur un châssis à l'intérieur duquel est aménagé un logement débouchant à l'extérieur du châssis par une ouverture aménagée sur le dessus du châssis. La structure de guidage permet le déplacement du générateur par rapport au châssis, entre une position de retrait dans laquelle le générateur est situé à l'intérieur du logement et une position de traitement dans laquelle le générateur est sorti du logement en passant par l'ouverture.

Le châssis porteur du générateur d'ondes de pression est monté coulissant sur un chariot mobile sur le châssis de l'appareil de traitement. Les mouvements combinés du chariot, du châssis et de la structure de guidage permettent de positionner le générateur d'ondes de pression dans sa position de traitement. En pratique, la cinématique de ce générateur d'ondes de pression apparaît relativement encombrante et complexe à mettre en oeuvre. Par ailleurs, si le générateur d'ondes de pression est à même d'occuper une position escamotée à l'intérieur du châssis, il doit être noté que ce générateur d'ondes de pression ne se trouve pas protéger contre des agressions susceptibles d'intervenir à travers l'ouverture de passage aménagée sur le dessus du châssis.

La présente invention vise donc à remédier aux inconvénients de l'état de la technique en proposant un nouvel appareil de traitement par ondes de pression, conçu pour permettre au générateur d'ondes de pression d'être positionné facilement dans sa position de traitement et une position escamotée retiré facilement par rapport à la table de traitement tout en garantissant sa totale protection contre notamment des heurts ou des chocs en position escamotée.

Un autre objet de l'invention est de proposer un appareil de traitement de faible encombrement conservant une facilité de mise en oeuvre et présentant un coût réduit.

Pour atteindre de tels objectifs, l'objet de l'invention concerne un appareil de traitement par ondes de pression, comportant un générateur d'ondes de pression, monté par l'intermédiaire d'une structure de guidage sur un châssis à l'intérieur duquel est aménagé un logement débouchant à l'extérieur du châssis par une ouverture du générateur, la structure de guidage étant montée sur le châssis pour s'inscrire dans le logement et permettre le déplacement du générateur par rapport au châssis, entre une position de retrait dans laquelle le générateur est situé à l'intérieur du logement et une position de traitement dans laquelle le générateur est sorti du logement par l'ouverture.

Selon l'invention, le générateur est monté à l'extrémité d'un bras supporté par la structure de guidage et le logement contient, en position de retrait, complètement le bras et le générateur, un écran d'affichage étant monté dans un couvercle supporté par une structure de déplacement apte à placer le couvercle dans une position de fermeture de l'ouverture, et dans une position en dehors de l'ouverture pour autoriser la sortie du générateur

De plus, l'appareil de traitement selon l'invention peut présenter en outre en combinaison au moins l'une et/ou l'autre des caractéristiques additionnelles suivantes :
- la structure de guidage comporte des moyens pour guider le générateur selon une trajectoire linéaire à l'intérieur du logement et jusqu'à son positionnement devant l'ouverture, et selon une trajectoire courbe à l'extérieur du logement,
- la structure de guidage comporte un chariot monté mobile linéairement sur le châssis et sur lequel le bras est monté selon une liaison pivot, un système came - élément suiveur étant interposé entre le bras et le châssis pour définir la trajectoire du bras,
- la came est montée de façon amovible sur le châssis tandis que l'élément suiveur est monté sur le bras,
- la structure de guidage comporte un dispositif de verrouillage du bras dans des positions fixes le long de sa trajectoire correspondant à la position de traitement, à la position de retrait et à une position intermédiaire entre ces positions de retrait et de traitement,
- le dispositif de verrouillage comporte un doigt de verrouillage piloté en déplacement linéaire par un organe de commande et destiné à coopérer en position de verrouillage avec des trous aménagés sur la came,
- les trous sont aménagés sur la came afin de correspondre respectivement aux positions de traitement, intermédiaire et de retrait du générateur,
- un capteur de position est adapté pour détecter lorsque le bras occupe la position de traitement,
- le bras présente un profil courbe,
- un dispositif bloque le bras en position de retrait,
- en position de fermeture de l'ouverture, l'écran est dirigé vers le logement et en position en dehors de l'ouverture, l'écran est dirigé en direction opposée du châssis.
- la structure de déplacement comporte au moins un bras de support du couvercle, relié au châssis à l'aide d'une articulation permettant une rotation du bras de support sur lui-même et son abaissement ou son soulèvement,
- un système de blocage du couvercle sur le châssis, en position de fermeture de l'ouverture.

Diverses autres caractéristiques ressortent de la description faite ci-dessous en référence aux dessins annexés qui montrent, à titre d'exemples non limitatifs, des formes de réalisation de l'objet de l'invention.
Les **Figures 1** et **2** sont des vues en perspective montrant un exemple de réalisation d'un appareil de traitement conforme à l'invention avec le générateur d'ondes de pression respectivement en position de traitement et en position de retrait.
La **Figure 3** est une vue en perspective montrant la structure de guidage du générateur d'ondes de pression conforme à l'invention.
Les **Figures 4A, 4B, 4C** sont des vues en plan montrant trois positions caractéristiques du générateur d'ondes de pression respectivement en position de traitement, en position intermédiaire et en position de retrait.
La **Figure 4D** illustre un exemple de réalisation d'une came faisant partie de la structure de guidage du générateur de l'appareil conforme à l'invention.
La **Figure 5** est une vue en coupe prise sensiblement selon les lignes V-V de la Fig. 4A.
La **Figure 6** est une vue en perspective d'un appareil de traitement conforme à l'invention avec l'ouverture de sortie du générateur d'ondes de pression complètement fermée par un couvercle.

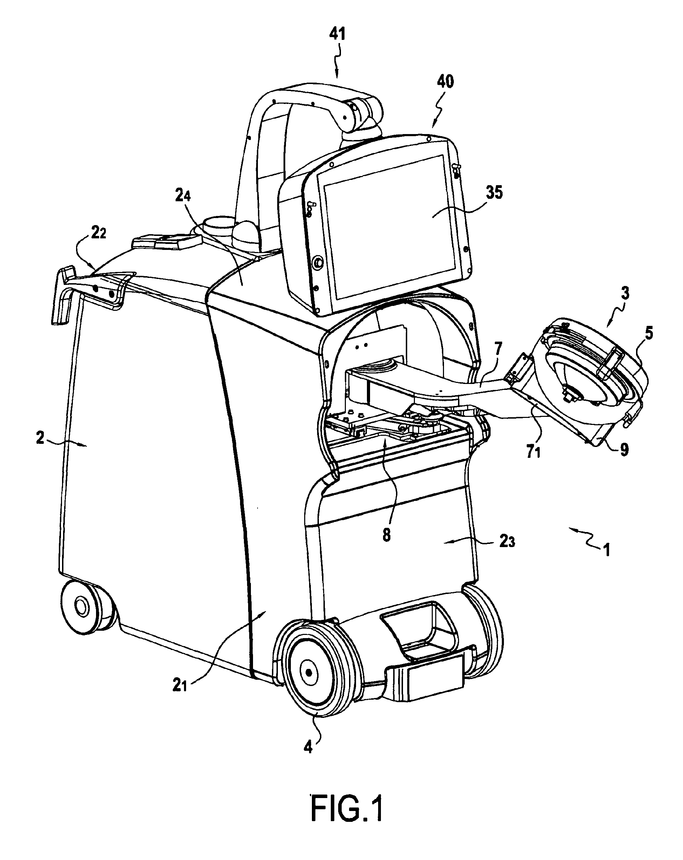

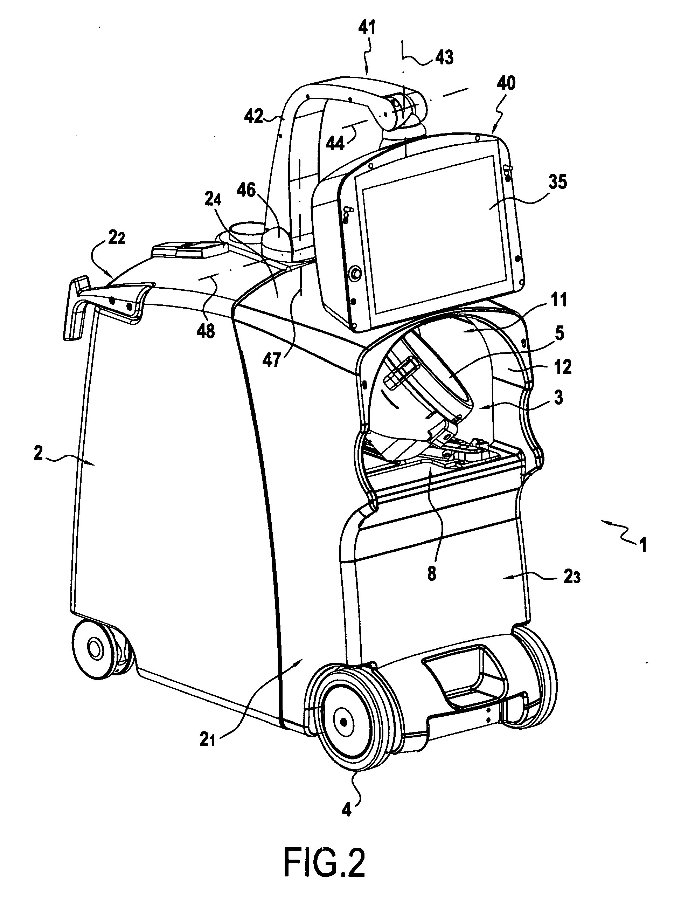

Tel que cela ressort plus précisément des **Fig. 1** et **2**, l'objet de l'invention concerne un appareil de traitement **1** par ondes de pression, de concrétions intracorporelles localisées dans le corps d'un patient ou d'un animal, à l'aide d'un système d'imagerie non décrit mais connu en soi. L'appareil de traitement **1** selon l'invention tel qu'un appareil de lithotritie, comporte un bâti ou un châssis **2** équipé d'un générateur **3** d'ondes de pression telles que des ondes de choc acoustiques.

Dans l'exemple de réalisation illustré, le châssis **2** présente une forme sensiblement parallélépipédique et se trouve avantageusement enveloppé par un capotage ou des parois de protection. Par exemple, le châssis **2** est ainsi équipé de deux parois de côté **2₁** s'étendant sensiblement parallèlement entre elles et reliées entre elles d'un côté par une paroi arrière **2₂** et de l'autre côté par une paroi avant **2₃**. Les parois de côté **2₁** et les parois avant **2₃** et arrière **2₂** sont réunies entre elles à leurs parties supérieures, par une paroi de dessus **2₄**. De manière classique, le châssis **2** est équipé de roues **4** permettant le roulement et le déplacement du châssis **2**.

De façon classique, le générateur **3** est adapté pour émettre des ondes de choc acoustiques dirigées vers un foyer cible que l'on cherche à mettre en coïncidence par l'intermédiaire d'un système d'imagerie, avec les concrétions en vue de détruire ces dernières. De manière conventionnelle, le générateur **3** comporte un réflecteur ellipsoïdal **5** dans lequel est positionnée une électrode qui permet de générer une onde de choc acoustique au premier foyer du réflecteur ellipsoïdal **5**. Par la géométrie particulière du réflecteur **5**, l'onde de choc produit au premier foyer se trouve réfléchie au second foyer du réflecteur, le second foyer se trouvant à l'extérieur du générateur, ce qui permet de viser des concrétions situées dans le corps d'un patient ou d'un animal. Le générateur **3** n'est pas décrit plus précisément car il ne fait pas partie précisément de l'invention et sa réalisation est bien connue de l'homme du métier.

Le générateur **3** est monté à l'extrémité distale **7₁** d'un bras **7** monté sur le châssis **2** à l'aide d'une structure de guidage **8** permettant le déplacement du générateur **3** entre une position de traitement illustrée à la **Fig. 1** dans laquelle le générateur **3** se trouve dans la position la plus éloignée du châssis **2** et une position de retrait illustrée à la **Fig. 2** dans laquelle le générateur **3** est rentré à l'intérieur du châssis **2.** Le générateur **3** est monté à l'extrémité distale **7₁** du bras **7,** par l'intermédiaire d'une liaison pivot **9**, permettant une orientation du générateur **3** par rapport au bras **7**.

Conformément à l'invention, le châssis **2** est aménagé pour délimiter intérieurement, un logement **11** débouchant à l'extérieur du châssis **2** par une ouverture **12.** Dans l'exemple de réalisation illustré, l'ouverture **12** est aménagée dans la paroi avant **2₃** du châssis s'étendant dans un plan sensiblement vertical. L'ouverture **12** est aménagée pour permettre la sortie du bras **7** et du générateur **3** afin que ce dernier atteigne sa position de traitement (**Fig. 1**) dans laquelle le générateur **3** est situé à l'extérieur du châssis **2.** Le logement **11** est aménagé pour en position de retrait, contenir complètement le bras **7** et le générateur **3 (****Fig.2****).**

Pour permettre la sortie et la rentrée du bras **7,** la structure de guidage **8** s'inscrit dans le logement **11,** c'est-à-dire qu'elle se trouve montée à l'intérieur du logement **11** pour permettre le déplacement du bras **7** et du générateur **3** entre sa position de retrait et sa position de traitement. Il est à noter que dans sa position de retrait, le générateur **3** se trouve complètement situé à l'intérieur du châssis **2** de manière à être protégé contre tout heurt ou choc. Dans l'exemple illustré, le châssis **2** est pourvu de parois de protection **2₁, 2₂, 2₃, 2₄** protégeant complètement l'appareil **1** mais il est clair que le châssis **2** peut être dépourvu partiellement ou complètement de parois de protection tout en assurant sa fonction de protection du générateur **3**. Selon cette variante de réalisation, le logement **11** est réalisé à l'intérieur d'un châssis plus ou moins ouvert en étant délimité par des montants et/ou traverses.

De préférence, la trajectoire du générateur **3** à l'intérieur du châssis **2** est optimisée pour limiter la taille du logement **11** et par suite l'encombrement de l'appareil **1**. Ainsi, la structure de guidage **8** comporte des moyens pour guider le générateur **3** selon une trajectoire linéaire à l'intérieur du logement **11** et jusqu'à son positionnement au niveau de l'ouverture **12**. Par ailleurs, la structure de guidage **8** comporte des moyens permettant de guider le générateur **3** selon une trajectoire courbe entre l'ouverture **12** et la position de traitement illustrée à la **Fig. 1**. Cette trajectoire courbe est avantageusement conçue pour permettre la mise en place et le retrait du générateur **3** à l'intérieur de la découpe aménagée dans la table de traitement tout en permettant d'éviter le système d'imagerie.

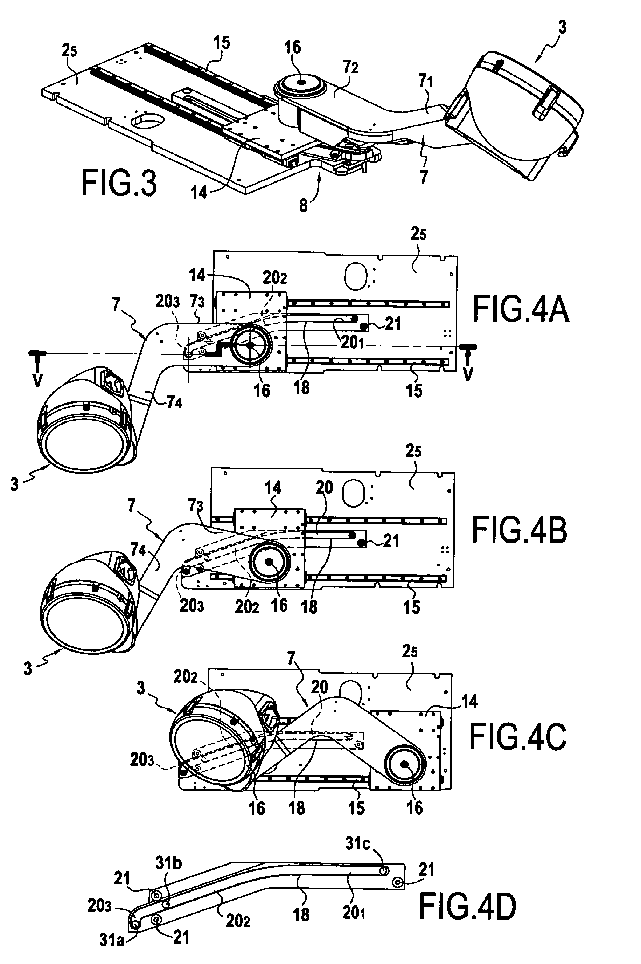

Tel que cela apparaît plus précisément aux **Fig. 3** à **5****,** la structure de guidage **8** comporte un chariot **14** adapté pour supporter le bras **7**. Le chariot **14** est monté mobile linéairement par rapport au châssis **2.** Dans l'exemple illustré, le chariot **14** est déplacé manuellement mais il est clair que son déplacement peut être motorisé. De préférence, le chariot **14** est monté mobile linéairement sur un jeu de glissières **15** fixées sur une platine **2₅** faisant partie du châssis **2**. Ces glissières rectilignes **15** permettent au chariot **14** de posséder une trajectoire linéaire à l'intérieur du logement **11**.

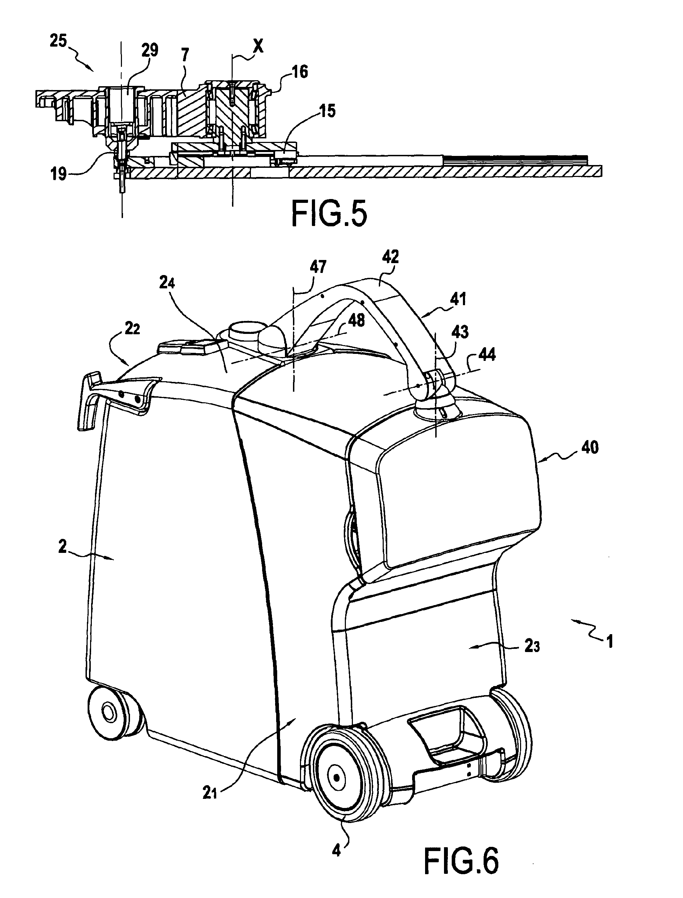

Le bras **7** est monté sur le chariot **14** selon une liaison pivot **16**. Tel que cela ressort plus précisément des **Fig. 3** et **5****,** la liaison pivot **16** est aménagée à une extrémité distale **7₂** du bras **7** s'étendant à l'opposé de l'extrémité distale **7₁** équipée du générateur **3**. La liaison pivot **16** est réalisée par tout moyen approprié permettant d'obtenir une rotation du bras **7** par rapport au chariot **14** et autour d'un axe vertical **X**.

Selon une variante préférée de réalisation, le bras **7** présente une forme coudée. Ainsi, tel que cela ressort plus précisément de la **Fig. 4A****,** le bras **7** comporte à partir de son extrémité proximale **7₂**, un premier segment droit **7₃** prolongé sensiblement à l'équerre par un deuxième segment droit **7₄** à l'extrémité duquel est fixé le générateur **3**.

La structure de guidage **8** comporte également un système came **18** et élément suiveur **19** interposé entre le bras **7** et le châssis **2** permettant de définir la trajectoire du bras **7.** Dans l'exemple de réalisation illustré, la came **18** est montée sur le châssis **2** (plus précisément sur la platine **2₅**) tandis que l'élément suiveur **19** est monté solidaire du bras **7.** Dans l'exemple illustré, la came **18** est réalisée par l'intermédiaire du profil d'une gorge **20**. De préférence, la came **18** est montée de manière amovible sur le châssis **2** par des vis **21** permettant son changement par une autre came présentant une gorge de profil différent conférant ainsi une trajectoire différente au bras **7**.

L'élément suiveur **19** est réalisé sous la forme d'un doigt engagé à l'intérieur de la rainure **20** de la came. L'élément suiveur **19** suit le profil de la came **18** lorsque le chariot **14** est déplacé linéairement le long des glissières **15.** La cinématique du bras **7** correspond au profil de la came **18** rendue possible par la combinaison du mouvement linéaire du chariot et du mouvement de rotation du bras **7** autour de la liaison pivot **16.** Ainsi, toute la complexité de la cinématique du bras **7** et par suite, du générateur **3** est ramenée à la réalisation du profil de la came **18**.

Dans l'exemple de réalisation illustré aux **Fig. 4A** à **4C**, la came **18** présente un premier segment **20₁** sensiblement parallèle à la glissière **15** et correspondant à la course linéaire du générateur **3** à l'intérieur du logement. Le premier segment **20₁** dont l'extrémité définit la position de retrait (**Fig. 4C**) est prolongé par un deuxième segment rectiligne **20₂** faisant une première angulation par rapport au premier segment **20₁**. Le deuxième segment **20₂** se trouve prolongé par un troisième segment **20₃** qui s'étend selon une direction sensiblement perpendiculaire par rapport au premier segment **20₁**. L'extrémité du troisième segment **20₃** définit la position de traitement illustrée à la **Fig. 4A** pour le générateur **3**. Bien entendu, la came **18** peut présenter un profil différent en fonction de la trajectoire souhaitée pour le générateur **3**.

De façon avantageuse, la position relative entre la came **18** et la liaison pivot **16** est choisie pour qu'en position de retrait (**Fig. 4C**), le bras **7** présente un encombrement limité. Ainsi, tel que cela ressort plus précisément de la **Fig. 4C**, la came **18** et la liaison pivot **16** sont montés relativement entre eux de manière que lors du déplacement du chariot **14** suivant le premier segment rectiligne **20₁** des glissières **15,** le bras **7** s'étend de manière sensiblement symétrique par rapport à la direction linéaire de déplacement du chariot **14.**

La structure de guidage **8** comporte un dispositif **25** de verrouillage du bras **7** dans des positions choisies ou prédéterminées le long de sa trajectoire définie par la came **18**. Selon une variante avantageuse de réalisation, les positions fixes du bras **7** correspondent à la position de traitement (**Fig. 4A**), à la position de retrait (**Fig. 4C**) et à une position intermédiaire (**Fig. 4B**) entre la position de retrait et la position de traitement. Cette position intermédiaire correspond par exemple à un recul limité du générateur **3** par rapport au générateur en position de traitement permettant par exemple d'accéder plus facilement au patient par exemple pour placer sur le patient, le gel de couplage acoustique des ondes de pression.

Dans l'exemple de réalisation illustré, le dispositif de verrouillage **25** est réalisé par le doigt suiveur **19** piloté en déplacement linéaire par un organe de commande **29** de manière à coopérer en position de verrouillage avec des trous **31a, 31b, 31c** aménagés sur la came **18**. Cet organe de commande **29** est de tout type connu en soi et peut être du type électromécanique commandé par l'utilisateur lorsque le générateur **3** occupe l'une des trois positions caractéristiques définies ci-dessus. Les trous **31a, 31b, 31c** sont aménagés sur le trajet de la came **18** de manière que le doigt suiveur **19** puisse coopérer avec chacun d'eux de manière que le bras **7** puisse occuper respectivement :
- la position de traitement avec le bras **7** complètement sorti **(****Fig. 4A****),** et le générateur **3** situé à l'extérieur du châssis **2,**
- la position intermédiaire correspondant à un escamotage partiel du générateur **3** pour faciliter l'accès au patient **(****Fig. 4B****),**
- la position de retrait ou d'escamotage total du générateur 3, utilisée lors de la réalisation d'actes d'endourologie ou lors du transport ou du stockage de l'appareil **1** (**Fig. 4C**).

Dans l'exemple illustré, les trous **31a** et **31c** sont aménagés respectivement aux extrémités respectivement du troisième segment **20₃** et du premier segment **20₁** alors que le trou **31b** est aménagé sur le deuxième segment **20₂**. Lorsque le doigt suiveur **19** est engagé à l'intérieur de l'un de ces trous **31a, 31b, 31c,** le bras **7** occupe une position fixe. Selon une variante avantageuse de réalisation, il est à noter que l'appareil de traitement **1** comporte un dispositif de blocage du bras **7** lorsque ce dernier occupe sa position de retrait. Ce dispositif de blocage de type mécanique assure la fixation du bras **7** par rapport au châssis notamment lors des opérations de transport de l'appareil. Par exemple, ce dispositif de blocage comporte une tige filetée s'étendant à partir du chariot **14** pour venir traverser une plaque du châssis **2** et sur laquelle est vissé un écrou de serrage.

Selon une caractéristique avantageuse de réalisation, l'appareil de traitement 1 comporte un capteur de position adapté pour détecter lorsque le bras **7** occupe la position de traitement. Par exemple, ce capteur de position détecte la présence de l'élément suiveur **19** lorsqu'il se trouve au niveau du trou **31a.**

Selon une autre caractéristique avantageuse de réalisation illustrée à la **Fig. 1** et à la **Fig. 2****,** l'appareil de traitement **1** comporte un écran d'affichage **35** du type par exemple LCD permettant la visualisation et/ou la commande dans le cadre du fonctionnement de l'appareil **1**. Cet écran **35** est monté dans un couvercle **40** qui est supporté par une structure de déplacement **41**. La structure de déplacement **41** est apte à placer le couvercle **40** dans une position de fermeture **(****Fig. 6****)** ou d'ouverture (**Fig. 2**) du logement **11.** Le couvercle **40** présente une section au moins égale voire légèrement supérieure à la section de l'ouverture **12** du logement pour permettre sa parfaite obturation ou fermeture et la protection du générateur **3** placé à l'intérieur du logement **11**.

Lorsque le couvercle **40** occupe la position de fermeture du logement **11,** l'écran **35** est selon une variante avantageuse mais non exclusive, dirigé vers l'intérieur du logement **11** pour être protégé. Selon une variante préférée de réalisation, l'appareil comporte un système non représenté permettant de bloquer le couvercle **40** sur le châssis **2**, en position de fermeture de l'ouverture **12**. Ainsi, le couvercle **40** se trouve fixé par exemple à l'aide de crochets, sur le châssis évitant tout mouvement intempestif susceptible d'endommager l'écran **35** lors du transport de l'appareil **1**. Dans cette position de fermeture, l'appareil **1** présente un encombrement limité et une forme compacte (**Fig. 6**).

Lorsque le couvercle **40** occupe une position située en dehors de l'ouverture **12** pour autoriser la sortie du générateur **3**, l'écran **35** est dirigé avantageusement, en direction opposée du châssis **2** pour permettre aux utilisateurs de visualiser l'écran lors de l'utilisation de l'appareil **1**. Cette structure de déplacement **41** comporte comme cela ressort plus précisément des **Fig. 2** et **6**, au moins un bras de support **42** à l'extrémité duquel est monté le couvercle **40,** à l'aide d'une part, d'un axe de rotation **43** vertical permettant de pivoter le couvercle **40** sur lui-même, selon un axe vertical de manière à placer l'écran **35** soit en regard du châssis, soit en direction opposée du châssis et d'autre part, d'au moins un axe de rotation horizontal **44** permettant de lever et/ou d'abaisser le couvercle par rapport à l'ouverture **12**. Le bras de support **42** est relié au châssis **2** à l'aide d'une articulation **46** permettant une rotation du bras de support **42** sur lui-même selon un axe vertical **47** et l'abaissement ou le soulèvement du bras de support **42** selon un axe horizontal **48**. La rotation du bras de support **42** selon l'axe horizontal **48** participe à la fermeture du logement **11** par le couvercle **40.** Par ailleurs, la rotation du bras de support **42** selon l'axe vertical **47** permet de positionner l'écran d'affichage **35** sur l'un des côtés du châssis **2** pour faciliter l'accès à l'écran d'affichage **35** et sa lecture. Dans l'exemple illustré, la structure de déplacement **41** comporte un bras unique mais il est clair que cette structure de déplacement peut comporter plusieurs segments ou bras articulés entre eux.

L'invention n'est pas limitée aux exemples décrits et représentés car diverses modifications peuvent y être apportées sans sortir de son cadre.

## Revendications

1. Appareil de traitement par ondes de pression, comportant un générateur d'ondes de pression (**3**), monté par l'intermédiaire d'une structure de guidage (**8**) sur un châssis (**2**) à l'intérieur duquel est aménagé un logement (**11**) débouchant à l'extérieur du châssis (**2**) par une ouverture (**12**) du générateur (**3**), la structure de guidage (**8**) étant montée sur le châssis pour s'inscrire dans le logement (**11**) et permettre le déplacement du générateur (**3**) par rapport au châssis (**2**), entre une position de retrait dans laquelle le générateur (**3**) est situé à l'intérieur du logement (**11**) et une position de traitement dans laquelle le générateur (**3**) est sorti du logement (**11**) par l'ouverture (**12**), **caractérisé en ce que** le générateur (**3**) est monté à l'extrémité d'un bras (**7**) supporté par la structure de guidage (**8**) et **en ce que** le logement (**11**) contient, en position de retrait, complètement le bras (**7**) et le générateur (**3**), un écran d'affichage (**35**) étant monté dans un couvercle (**40**) supporté par une structure de déplacement (**41**) apte à placer le couvercle (**40**) dans une position de fermeture de l'ouverture (**12**), et dans une position en dehors de l'ouverture (**12**) pour autoriser la sortie du générateur (**3**).

2. Appareil de traitement selon la revendication 1, **caractérisé en ce que** la structure de guidage (**8**) comporte des moyens pour guider le générateur (**3**) selon une trajectoire linéaire à l'intérieur du logement (**11**) et jusqu'à son positionnement devant l'ouverture (**12**), et selon une trajectoire courbe à l'extérieur du logement (**11**).

3. Appareil de traitement selon la revendication 1 ou 2, **caractérisé en ce que** la structure de guidage (**8**) comporte un chariot (**14**) monté mobile linéairement sur le châssis et sur lequel le bras (**7**) est monté selon une liaison pivot (**16**), un système came (**18**) - élément suiveur (**19**) étant interposé entre le bras (**7**) et le châssis (**2**) pour définir la trajectoire du bras.

4. Appareil de traitement selon la revendication 3, **caractérisé en ce que** la came (**18**) est montée de façon amovible sur le châssis (**2**) tandis que l'élément suiveur (**19**) est monté sur le bras (**7**).

5. Appareil de traitement selon l'une des revendications 1 à 4, **caractérisé en ce que** la structure de guidage (**8**) comporte un dispositif de verrouillage (**25**) du bras (**7**) dans des positions fixes le long de sa trajectoire correspondant à la position de traitement, à la position de retrait et à une position intermédiaire entre ces positions de retrait et de traitement.

6. Appareil de traitement selon la revendication 5, **caractérisé en ce que** le dispositif de verrouillage (**25**) comporte un doigt de verrouillage (**19**) piloté en déplacement linéaire par un organe (**29**) de commande et destiné à coopérer en position de verrouillage avec des trous aménagés (**31a**, **31b**, **31c**) sur la came (**18**).

7. Appareil de traitement selon la revendication 6, **caractérisé en ce que** les trous (**31a**, **31b**, **31c**) sont aménagés sur la came (**18**) afin de correspondre respectivement aux positions de traitement, intermédiaire et de retrait du générateur (**3**).

8. Appareil de traitement selon la revendication 6 ou 7, **caractérisé en ce qu'**il comporte un capteur de position adapté pour détecter lorsque le bras (**7**) occupe la position de traitement.

9. Appareil de traitement selon l'une des revendications 1 à 8, **caractérisé en ce que** le bras (**7**) présente un profil courbe.

10. Appareil de traitement selon l'une des revendications 1 à 9, **caractérisé en ce qu'**il comporte un dispositif de blocage du bras (**7**) en position de retrait.

11. Appareil de traitement selon la revendication 1, **caractérisé en ce qu'**en position de fermeture de l'ouverture (**12**), l'écran (**35**) est dirigé vers le logement (**11**) et qu'en position en dehors de l'ouverture (**12**), l'écran (**35**) est dirigé en direction opposée du châssis (**2**).

12. Appareil de traitement selon la revendication 1, **caractérisé en ce que** la structure de déplacement (**41**) comporte au moins un bras de support (**42**) du couvercle (**40**), relié au châssis (**2**) à l'aide d'une articulation (**46**) permettant une rotation du bras de support (**42**) sur lui-même et son abaissement ou son soulèvement.

13. Appareil de traitement selon la revendication 1, **caractérisé en ce qu'**il comporte un système de blocage du couvercle (**40**) sur le châssis (**2**), en position de fermeture de l'ouverture (**12**).

## Patentansprüche

1. Gerät zur Behandlung mittels Druckwellen, umfassend einen Druckwellengenerator (3), der über eine Führungsstruktur (8) an einem Rahmen (2) angebracht ist, in dem eine Aufnahme (11) eingerichtet ist, welche über eine Öffnung (12) des Generators (3) außerhalb des Rahmens (2) ausmündet, wobei die Führungsstruktur (8) an dem Rahmen angebracht ist, um sich in die Aufnahme (11) einzufügen und um das Bewegen des Generators (3) gegenüber dem Rahmen (2), zwischen einer Einzugsposition, in welcher der Generator (3) sich innerhalb der Aufnahme (11) befindet, und einer Behandlungsposition, in welcher der Generator (3) über die Öffnung (12) aus der Aufnahme (11) ausgefahren ist, zu ermöglichen, **dadurch gekennzeichnet, daß** der Generator (3) am Ende eines durch die Führungsstruktur (8) getragenen Arms (7) angebracht ist und daß die Aufnahme (11) in der Einzugsposition den Arm (7) und den Generator (3) vollständig enthält, wobei ein Bildschirm (35) in einem Deckel (40) angebracht ist, der von einer Bewegungsstruktur (41) getragen ist, welche geeignet ist, den Deckel (40) in eine Position zum Verschließen der Öffnung (12) und in eine Position außerhalb der Öffnung (12), um das Ausfahren des Generators (3) zuzulassen, zu bewegen.

2. Behandlungsgerät nach Anspruch 1, **dadurch gekennzeichnet, daß** die Führungsstruktur (8) Mittel umfaßt, um den Generator (3) entlang einer linearen Bahn innerhalb der Aufnahme (11) und bis zu seiner Positionierung vor der Öffnung (12) sowie entlang einer gekrümmten Bahn außerhalb der Aufnahme (11) zu führen.

3. Behandlungsgerät nach Anspruch 1 oder 2, **dadurch gekennzeichnet, daß** die Führungsstruktur (8) einen Schlitten (14) umfaßt, der an dem Rahmen linear beweglich angebracht ist und an dem der Arm (7) um eine Drehgelenkverbindung (16) angebracht ist, wobei ein Kurve (18) / Nachlaufelement (19) - System zwischen dem Arm (7) und dem Rahmen (2) eingefügt ist, um die Bahn des Arms zu definieren.

4. Behandlungsgerät nach Anspruch 3, **dadurch gekennzeichnet, daß** die Kurve (18) an dem Rahmen (2) lösbar angebracht ist, während das Nachlaufelement (19) an dem Arm (7) angebracht ist.

5. Behandlungsgerät nach einem der Ansprüche 1 bis 4, **dadurch gekennzeichnet, daß** die Führungsstruktur (8) eine Vorrichtung zur Verrieglung (25) des Arms (7) in festen Positionen entlang seiner Bahn, die der Behandlungsposition, der Einzugsposition und einer Zwischenposition zwischen diesen Einzugs- und Behandlungspositionen entsprechen, umfaßt.

6. Behandlungsgerät nach Anspruch 5, **dadurch gekennzeichnet, daß** die Verriegelungsvorrichtung (25) einen Verriegelungsfinger (19) umfaßt, der durch ein Steuerorgan (29) linearbeweglich gesteuert wird und der dazu bestimmt ist, in der Verriegelungsposition mit an der Kurve (18) angeordneten Löchern (31a, 31b, 31c) zusammenzuwirken.

7. Behandlungsgerät nach Anspruch 6, **dadurch gekennzeichnet, daß** die Löcher (31 a, 31 b, 31c) an der Kurve (18) angeordnet sind, um der Behandlungsposition, der Zwischenposition bzw. der Einzugsposition des Generators (3) zu entsprechen.

8. Behandlungsgerät nach Anspruch 6 oder 7, **dadurch gekennzeichnet, daß** es einen Lagesensor umfaßt, der dazu ausgelegt ist, zu erfassen, wenn der Arm (7) die Behandlungsposition einnimmt.

9. Behandlungsgerät nach einem der Ansprüche 1 bis 8, **dadurch gekennzeichnet, daß** der Arm (7) ein gebogenes Profil aufweist.

10. Behandlungsgerät nach einem der Ansprüche 1 bis 9, **dadurch gekennzeichnet, daß** es eine Vorrichtung zum Festlegen des Arms (7) in der Einzugsposition umfaßt.

11. Behandlungsgerät nach Anspruch 1, **dadurch gekennzeichnet, daß** in der Position zum Verschließen der Öffnung (12) der Bildschirm (35) zu der Aufnahme (11) hin ausgerichtet ist und daß in der Position außerhalb der Öffnung (12) der Bildschirm (35) in von dem Rahmen (2) abgewandter Richtung ausgerichtet ist.

12. Behandlungsgerät nach Anspruch 1, **dadurch gekennzeichnet, daß** die Bewegungsstruktur (41) wenigstens einen Arm zum Tragen (42) des Deckels (40) umfaßt, der mit Hilfe eines Gelenks (46), das ein Drehen des Tragarms (42) um ihn selbst sowie sein Absenken oder sein Anheben ermöglicht, mit dem Rahmen (2) verbunden ist.

13. Behandlungsgerät nach Anspruch 1, **dadurch gekennzeichnet, daß** es ein System zum Festlegen des Deckels (40) an dem Rahmen (2) in der Position zum Verschließen der Öffnung (12) umfaßt.

## Claims

1. A pressure wave treatment appliance comprising a pressure wave generator (3) mounted via a guide structure (8) on a base member (2) halving a housing (11) arranged therein, the housing opening out to the outside of the base member (2) via an opening (12) of the generator (3), the guide structure (8) being mounted on the base member to be container swithin the housing (11) and to allow the generator (3) to move relative to the base member (2) between a retracted position in which the generator (3) is situated inside the housing (11) and a treatment position in which the generator (3) is extended from the housing (11) via the opening (12), the appliance being **characterized in that** the generator (3) is mounted at the end of an arm (7) supported by the guide structure (8), and **in that**, in the retracted position, the housing (11) contains the arm (7) and the generator (3) completely, a display screen (35) being mounted in a cover (40) supported by a movement structure (41) suitable for placing the cover (40) in a position for closing the opening (12), and in a position away from the opening (12) in order to allow the generator (3) to be extracted.

2. A treatment appliance according to claim 1, **characterized in that** the guide structure (8) includes means for guiding the generator (3) along a linear path inside the housing (11) until it is positioned in front of the opening (12), and along a curved patch outside the housing (11).

3. A treatment appliance according to claim 1 or claim 2, **characterized in that** the guide structure (8) includes a carriage (14) mounted to move linearly on the base member and halving the arm (7) mounted thereon via a pivot connection (16), a system comprising a cam (18) and a cam follower (19) being interposed between the arm (7) and the base member (2) to define the path of the arm.

4. A treatment appliance according to claim 3, **characterized in that** the cam (18) is removably mounted on the base member (2), while the cam follower (19) is mounted on the arm (7).

5. A treatment appliance according to any one of claims 1 to 4, **characterized in that** the guide structure (8) includes a locking device (25) for locking the arm (7) in fixed positions along its path corresponding to the treatment position, to the retracted position, and to an intermediate position between said retracted and treatment positions.

6. A treatment appliance according to claim 5, **characterized in that** the locking device (25) includes a locking finger (19) controlled two move in linear manner by a control member (29) and serving, in the locking position, to co-operate with holes (31a, 31b, 31c) formed in the cam (18).

7. A treatment appliance according to claim 6, **characterized in that** the holes (31a, 31b, 31c) are arranged in the cam (18) so as to correspond respectively with the treatment, intermediate, and retracted positions of the generator (3).

8. A treatment appliance according to claim 6 or claim 7, **characterized in that** it includes a position sensor adapted to detect when the arm (7) is occupying the treatment position.

9. A treatment appliance according to any one of claims 1 to 8, **characterized in that** the arm (7) presents a curved profile.

10. A treatment appliance according to any one of claims 1 to 9, **characterized in that** it includes a device for blocking the arm (7) in the retracted position.

11. A treatment appliance according to claim 1, **characterized in that**, in its position for closing the opening (12), the screen (35) faces towards the housing (11), and in its position outside the opening (12), the screen (35) faces away from the base member (2).

12. A treatment appliance according to claim 1, **characterized in that** the movement structure (41) includes at least one support arm (42) for supporting the cover (40) and connected to the base member (2) via a hinge (46) enabling the support arm (42) to swivel and enabling it to be lowered or raised.

13. A treatment appliance according to claim 1, **characterized in that** it includes a system for blocking the cover (40) on the base member (2) in its position closing the opening (12).
